# EUROPÄISCHE PATENTSCHRIFT

(11) **EP 1 753 477 B1**
(45) Veröffentlichungstag und Bekanntmachung des Hinweises auf die Patenterteilung: **21.05.2008**
(21) Anmeldenummer: 05739702.8
(22) Anmeldetag: 12.04.2005
(51) Int. Cl.: A61L 27/10, A61F 2/36

(54) **HÜFTGELENK-ENDOPROTHESE MIT HÜLSE AUS BIOKOMPATIBLEM KUNSTSTOFF**
HIP JOINT ENDOPROSTHESIS HAVING A SLEEVE FROM A BIOCOMPATIBLE PLASTIC
ENDOPROTHESE DE L'ARTICULATION DE LA HANCHE, POURVUE D'UN MANCHON EN PLASTIQUE BIOCOMPATIBLE

(30) Priorität: 07.06.2004 DE 102004027658
(43) Veröffentlichungstag der Anmeldung: 21.02.2007
(73) Patentinhaber: Mathys AG Bettlach, 2544 Bettlach (CH)
(72) Erfinder: COTTING, Anton, CH-2540 Grenchen (CH); SALOMON, Dirk, 04626 Jonaswalde (DE); GLIEN, Wilfried, 07639 Bad Klosterlausnitz (DE); OBERBACH, Thomas, 07629 Reichenbach (DE)
(74) Vertreter: Körfer, Thomas
(86) Internationale Anmeldenummer: PCT/EP2005/003845
(87) Internationale Veröffentlichungsnummer: WO 2005/120596

(56) Entgegenhaltungen:
- EP-A- 0 385 572
- EP-A- 0 768 066
- CH-A- 676 922
- DE-A1- 4 008 563
- DE-A1- 10 156 610
- DE-U1- 9 209 584

## Beschreibung

Die Erfindung betrifft eine Hüftgelenk-Endoprothese zur prothetischen Versorgung des menschlichen oder tierischen Hüftgelenks.

Beispielsweise ist aus der DE 101 56 610 A1 eine Gelenkkugel für eine Hüftgelenk-Endoprothese bekannt. Diese weist einen innenliegenden metallischen Armierungskörper auf, auf welchem eine Beschichtung als Gleitpartner für ihr Gegenstück im Gelenk ausgebildet ist. In dem metallischen Armierungskörper ist eine konische Klemmhülse zur Aufnahme eines Hüftschaftkonus angeordnet.

Nachteilig an der aus der DE 101 56 610 A1 bekannten Gelenkkugel ist insbesondere, daß die Klemmhülse lediglich der Fixierung des Hüftschaftkonus dient und Abweichungen des Hüftschaftkonus von der optimalen Form durch Beschädigungen oder Verunreinigungen der Oberfläche nicht ausgeglichen werden können.

Die Druckschrift DE 92 09 584.4 U1 geht aus von einer Hüftgelenkendoprothese, die aus einem Prothesenschaft, einem Prothesenhals und einem lösbaren Steckkopf besteht, wobei der Steckkopf mit einer Gleitvorrichtung versehen ist, welche eine integrierte Dämpfungsvorrichtung aufweist.

Weiterhin ist aus der CH 676 922 A5 eine Gelenkendoprothese mit einer Konus-Steckverbindung zwischen einem keramischen und einem metallischen Gelenkteil bekannt, bei welcher die Konus-Steckverbindung in eine metallische Zwischenhülse verlagert ist, die im keramischen Gelenkteil fest vormontiert wird. Intraoperativ wird dann nur noch die zweite Steckverbindung zusammengefügt.

Nachteilig an der aus der CH 676 922 A5 bekannten Gelenkendoprothese ist dabei insbesondere, daß eine metallische Zwischenhülse nur sehr unzureichend geeignet ist, Verformungen im Bereich der konischen Steckverbindung auszugleichen, welche beispielsweise durch Beschädigungen der konischen Flächen oder durch Verunreinigungen hervorgerufen werden.

Der Erfindung liegt demnach die Aufgabe zugrunde, eine Hüftgelenk-Endoprothese zu schaffen, welche so ausgebildet ist, daß Oberflächenunebenheiten durch Beschädigungen und Verunreinigungen kompensiert werden können und dadurch die Möglichkeit zum Austausch der Gelenkkugel geboten wird, ohne eine Totaloperation nötig zu machen.

Die Aufgabe wird durch die Merkmale des Anspruchs 1 gelöst.

Gemäß Anspruch 1 ist vorgesehen, eine Hülse zwischen einer Innenwandung der Gelenkkugel und dem Hüftschaftkonus anzuordnen, welche aus einem biokompatiblen Kunststoff hergestellt ist.

Weitere vorteilhafte Ausgestaltungen der Erfindung sind in den Unteransprüchen gekennzeichnet.

Von Vorteil ist, daß eine Hülse aus einem Polymer wie Polyethylen oder Polyetheretherketon thermoplast und gleichzeitig schlagzäh ist und dadurch beste Voraussetzungen zum Ausgleich von Beschädigungen und Verunreinigungen auf einer der Oberflächen der konischen Steckverbindung bietet.

Vorteilhafterweise weist die Hülse einen umlaufenden Wulst auf, der mit einer Nut der Gelenkkugel in Eingriff steht, wodurch die Hülse vormontiert mit der Gelenkkugel angeliefert werden kann, ohne daß die Gefahr des Herausrutschens beim Transport und während der Operation besteht.

Weiterhin ist von Vorteil, gemäß Anspruch 1, daß die Hülse becherförmig ausgebildet ist und in einem Boden eine Ausnehmung aufweist, durch welche Luft, die bei der Vormontage zwischen Hülse und Gelenkkugel eingeschlossen wird, entweichen kann.

Ein Ausführungsbeispiel der Erfindung wird im folgenden anhand teilweise schematischer Darstellungen in der Zeichnung näher erläutert. In der Zeichnung zeigen:
- Fig. 1: eine schematische Ansicht eines Ausführungsbeispiels einer erfindungsgemäß ausgestalteten Hüftgelenk-Endoprothese, und
- Fig. 2A-C: verschiedene vergrößerte Ansichten der erfindungsgemäß ausgestalteten Gelenkkugel der Hüftgelenk-Endoprothese gemäß Fig. 1.

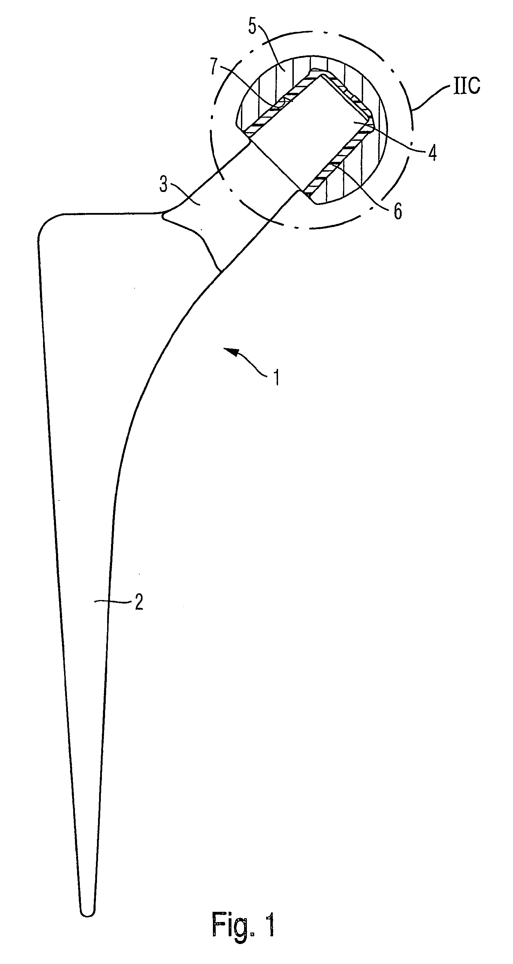

Fig. 1 zeigt in einer schematischen Darstellung eine Ansicht einer Femurkomponente 1 für eine Hüftgelenk-Endoprothese. Die im Ausführungsbeispiel dargestellte Hüftgelenk-Endoprothese wird auch als totale Hüftgelenk-Endoprothese bezeichnet, da sowohl der femurale als auch der beckenseitige Gelenkanteil ersetzt wird. Die Femurkomponente 1 weist einen Schaft 2 auf, welcher in der Markhöhle eines Femurknochens je nach Bauform der Femurkomponente 1 einzementiert oder zementfrei verankert wird. An dem Schaft 2 ist eine Halspartie 3 ausgebildet, an deren Ende ein Hüftschaftkonus 4 zur Aufnahme einer Gelenkkugel 5 ausgebildet ist.

Die im folgenden näher beschriebenen erfindungsgemäßen Maßnahmen sind unabhängig von der Form des Schaftes 2 sowie in der Kombination mit einer nicht weiter dargestellten Gelenkpfanne oder mit einem direkt in der Gelenkpfanne artikulierenden Prothesenkopfsystem zu sehen. Die Form der Gelenkpfanne ist beliebig. Auch die Form der Halspartie 3 ist frei wählbar.

Für die Gleitpaarungen zwischen der Gelenkkugel 5 und der Gelenkpfanne können verschiedene Materialien verwendet werden. Dies können beispielsweise Polyethylen (PE) mit einer Metallegierung wie Cobalt-Chrom (CoCr) oder mit Keramik sein, diese Paarung hat jedoch den Nachteil von starkem Abrieb und damit verbundenem schleichenden Verfall durch Einlagerungen des Abriebs im Pfannenhintergrund und Auslockerung der Pfanne. Gleitpaarungen aus zwei metallischen Komponenten sind ebenfalls nicht unkritisch, da der Abrieb und die anschließende Schwerionendiffusion in den Körper zu erhöhten Schwermetallwerten im Blutbild führt und zudem, insbesondere im Fall von Nickel, allergieauslösend wirken kann.

Keramische Gleitlager dagegen zeigen sehr geringen Abrieb, welcher zudem bioinert ist, und ermöglichen hochpräzise Oberflächenbearbeitung im Nano-Bereich. Die Verwendung von Gelenkkugeln 5 aus Keramik hat sich daher dank der guten tribologischen Eigenschaften in der Hüftendoprothetik durchgesetzt. Nachteilig ist hier jedoch die Gefahr eines plötzlichen Schadensfalls durch Bruch der Gelenkkugel 5, da die Belastbarkeit von Keramik auf Zugspannungen schlecht ist.

Zugspannungen können dabei bereits durch Verformungen des Hüftschaftkonus 4 entstehen, die im Mikrometer-Bereich liegen. Falls der Hüftschaftkonus 4 vor dem Aufsetzen eines Gelenkkopfsystems 5 aus Keramik nicht frei von Partikeln (Knochenreste, Blut, Fett, Weichteilen etc.) ist oder die Oberfläche des Hüftschaftkonus 4 nicht oder nicht mehr den Vorgaben entspricht, können in der Gelenkkugel 5 unerwünschte Zugspannungen auftreten und lokal zu Spannungsspitzen führen, die zum Bruch der Gelenkkugel 5 führen können. Dabei ist festzuhalten, daß es sich hierbei nicht um einen dynamischen Prozeß handelt, beispielsweise bei starken punktuellen Belastungen bei sportlicher Betätigung o.ä., sondern um ein aus dauernder statischer Belastung herrührendes Schädigungsbild.

Weiterhin kann bei der Revision der Gelenkkugel 5, bei welcher der Schaft 2 in situ verbleiben könnte, die Wahl nicht wieder auf eine Gelenkkugel 5 aus Keramik fallen, da der Hüftschaftkonus 4 durch das Entfernen der Gelenkkugel 5 möglicherweise beschädigt wurde und eine von der Ursprungsgeometie abweichende Konusgeometrie aufweist, was zu oben genannten Problemen analog zu den Verunreinigungen führen kann.

Sicherheit gegen einen Schadensfall durch Bruch der Gelenkkugel 5 aufgrund abweichender Geometrie des Hüftschaftkonus 4 kann somit nur eine Total-Operation gewährleisten, bei welcher nicht nur die Gelenkkugel 5, sondern die gesamte Femurkomponente 1 ausgetauscht wird, was jedoch gravierende Nachteile birgt. Einerseits ist der Schaft 2 oftmals gut in den Femurknochen integriert und kann nur unter hohem Aufwand entfernt werden, wobei zudem Knochengewebe des Femurs verlorengeht. Weiterhin ist die Operationsdauer bei einer Totaloperation erheblich länger als beim Austausch der Gelenkkugel 5. Zudem ist der für eine Totaloperation benötigte Gewebeschnitt größer und demnach auch die postoperative Infektionsgefahr höher und die Wahrscheinlichkeit der Bildung von Vernarbungen höher.

Aufgabe der vorliegenden Erfindung ist es demnach, die Gelenkkugel 5 so zu konzipieren, daß die keramischen Teile der Hüftgelenk-Endoprothese revisionsfähig werden, wodurch eine Totaloperation unnötig ist und eine neue keramische Gelenkkugel 5 auf den vorhandenen Schaft 2 aufgesetzt werden kann.

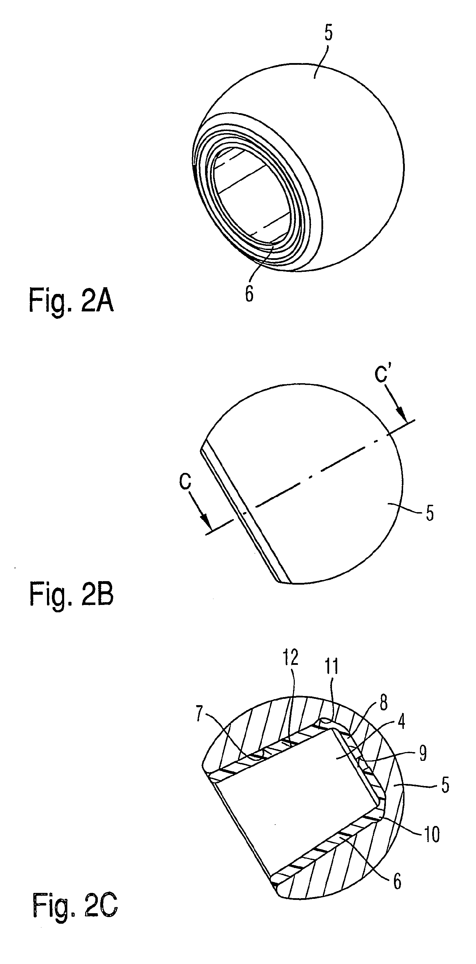

Erfindungsgemäß ist daher vorgesehen, wie in Fig. 2A bis 2C in verschiedenen Ansichten vergrößert dargestellt, in die keramische Gelenkkugel 5 eine Hülse 6 einzusetzen, welche die Fehlertoleranz vergrößert und das Risiko eines Bruchs der Gelenkkugel 5 reduziert. Die Hülse 6 ist dabei becherförmig, insbesondere in Form einer konischen Hülse 6 ausgebildet, die einen Boden 8 aufweist. Die Hülse 6 liegt an einer Innenwandung 7 der keramischen Gelenkkugel 5 an und wird dem Operateur mit diesem fest vormontiert geliefert. Der Operateur muß intraoperativ dann nur noch die Gelenkkugel 5 mit der eingelegten Hülse 6 auf den Hüftschaftkonus 4 aufstecken. Durch die weiter unten näher erläuterten Materialeigenschaften der Hülse 6 steht nicht mehr zu befürchten, daß Spannungsspitzen auftreten, welche zu einem plötzlichen Schadensfall führen können.

Zur Erleichterung der Vormontage weist die becherförmige Hülse 6, wie aus Fig. 2C ersichtlich, im Boden 8 eine Ausnehmung 9 auf, welche der bei der Montage zwischen der Hülse und der Innenwandung 7 der Gelenkkugel 5 eingeschlossenen Luft ermöglicht zu entweichen, so daß die Hülse 6 vollflächig an der Innenwandung 7 der Gelenkkugel 5 und an einer Außenfläche 12 des Hüftschaftkonus 4 anliegt. Dies ist eine wesentliche Voraussetzung für die korrekte Funktion der Hülse 6.

Um zu verhindern, daß sich die Hülse 6 während des Transports oder der Operation von der Gelenkkugel 5 löst, weist die Gelenkkugel 5 an der Innenwandung 7 eine umlaufende Nut 11 auf, mit welcher ein an der Hülse 6 ausgebildeter umlaufender Wulst 10 in Eingriff steht. Der Wulst 10 und die Nut 11 sind im Ausführungsbeispiel im Bereich des Bodens 8 der becherförmigen konischen Hülse 6 ausgebildet. Da die Herstellung hier besonders einfach ist, es ist jedoch auch möglich, den Wulst 10 und die Nut 11 in einer anderen Position anzubringen oder die Hülse 6 direkt ohne zusätzliche Nut einzubringen.

Die Hülse 6 besteht vorzugsweise aus einem biokompatiblen Material mit niedrigerer Steifigkeit und höherer Bruchzähigkeit als die Keramik der Gelenkkugel 5, insbesondere aus einem Polymer wie Polyetheretherketon (PEEK) oder Polyethylen (PE) oder einem beliebigen biokompatiblem Kunststoff, der durch seine thermoplasten, schlagzähen Eigenschaften eine pseudoelastische Verbindung zwischen den Prothesenkomponenten ermöglicht.

Die Wahl des Materials für die Hülse 6 weist dabei zwei wesentliche Vorteile auf: einerseits können Verunreinigungen oder Beschädigungen einer der Oberflächen der konischen Steckverbindung sehr effektiv ausgeglichen werden, so daß Schadensfälle durch Bruch der keramischen Gelenkkugel 5 vermieden werden. Andererseits ist es nicht mehr nötig, die Oberflächen der Steckverbindung hochpräzise zu bearbeiten, da geringfügige Abweichungen von der idealen Fläche ebenfalls ausgeglichen werden, was die Herstellungskosten erheblich senkt.

Die Erfindung ist nicht auf das dargestellte Ausführungsbeispiel beschränkt und auch für andere Formen von Femurkomponenten 1 anwendbar. Insbesondere ist die Anwendung der Hülse 6 nicht auf keramische Gelenkkugeln 5 beschränkt, sondern ist auch für die Anwendung beispielsweise mit einem keramisch ausgebildeten Hüftschaftkonus 4 geeignet.

## Patentansprüche

1. Hüftgelenk-Endoprothese mit einer Femurkomponente (1), die einen in einer Markhöhle eines Femurknochens verankerbaren Schaft (2) und eine daran ausgebildeten Halspartie (3) mit einem Hüftschaftkonus (4) zur Aufnahme einer keramischen Gelenkkugel (5) umfasst, wobei zwischen dem Hüftschaftkonus (4) und einer Innenwandung (7) der Gelenkkugel (5) eine Hülse (6) angeordnet ist, wobei die Hülse (6) aus einem biokompatiblen Kunststoff hergestellt ist und einen der Innenwandung (7) der Gelenkkugel (5) zugewandten Boden (8) aufweist,
**dadurch gekennzeichnet,**
**dass** die Hülse (6) becherförmig geformt ist und
**dass** in dem Boden (8) eine Ausnehmung (9) vorgesehen ist.

2. Hüftgelenk-Endoprothese nach Anspruch 1,
**dadurch gekennzeichnet,**
**dass** der biokompatible Kunststoff ein Polymer ist.

3. Hüftgelenk-Endoprothese nach Anspruch 2,
**dadurch gekennzeichnet,**
**dass** das Polymer Polyethylen (PE), Polyetheretherketon (PEEK) oder Polymethylmethacrylat (PMMA) ist.

4. Hüftgelenk-Endoprothese nach einem der Ansprüche 1 bis 3,
**dadurch gekennzeichnet,**
**dass** die Hülse (6) becherförmig konisch geformt ist.

5. Hüftgelenk-Endoprothese nach einem der Ansprüche 1 bis 4,
**dadurch gekennzeichnet,**
**dass** die Hülse (6) einen umlaufenden Wulst (10) aufweist.

6. Hüftgelenk-Endoprothese nach Anspruch 5,
**dadurch gekennzeichnet,**
**dass** der umlaufende Wulst (10) mit einer Nut (11) in Eingriff steht, die an der Innenwandung (7) der Gelenkkugel (5) ausgebildet ist.

7. Hüftgelenk-Endoprothese nach einem der Ansprüche 1 bis 6,
**dadurch gekennzeichnet,**
**dass** die Hülse (6) vollflächig an der Innenwandung (7) der Gelenkkugel (5) und an einer Außenfläche (12) des Hüftschaftkonus (4) anliegt.

## Claims

1. A hip-joint endoprosthesis with a femur component (1) which comprises a shank (2), which is capable of being anchored in a medullary cavity of a femoral bone, and a neck portion (3) formed thereon with a hip-shank cone (4) for receiving a ceramic joint ball (5), a sleeve (6) being arranged between the hip-shank cone (4) and an inner wall (7) of the joint ball (5), the sleeve (6) being produced from a biocompatible plastic and exhibiting a bottom (8) facing towards the inner wall (7) of the joint ball (5),
**characterised in that**
the sleeve (6) is shaped in the form of a cup and
**in that** a recess (9) is provided in the bottom (8).

2. Hip-joint endoprosthesis according to Claim 1,
**characterised in that**
the biocompatible plastic is a polymer.

3. Hip-joint endoprosthesis according to Claim 2,
**characterised in that**
the polymer is polyethylene (PE), polyether ether ketone (PEEK) or polymethyl methacrylate (PMMA).

4. Hip-joint endoprosthesis according to one of Claims 1 to 3,
**characterised in that**
the sleeve (6) is conically shaped in the form of a cup.

5. Hip-joint endoprosthesis according to one of Claims 1 to 4,
**characterised in that**
the sleeve (6) exhibits a peripheral bead (10).

6. Hip-joint endoprosthesis according to Claim 5,
**characterised in that**
the peripheral bead (10) is in engagement with a groove (11) which is formed on the inner wall (7) of the joint ball (5).

7. Hip-joint endoprosthesis according to one of Claims 1 to 6,
**characterised in that**
the sleeve (6) bears over its full surface against the inner wall (7) of the joint ball (5) and against an external surface (12) of the hip-shank cone (4).

## Revendications

1. Endoprothèse de l'articulation de la hanche avec un composant fémur (1) qui comprend une hampe (2) pouvant être ancrée dans une cavité médullaire d'un os de fémur et une partie de col (3) formée sur la hampe avec un cône de hampe de hanche (4) pour recevoir une sphère d'articulation (5) en céramique, dans laquelle, entre le cône de hampe de hanche (4) et une paroi interne (7) de la sphère d'articulation (5), est agencé un manchon (6), le manchon (6) étant fabriqué à partir d'une matière synthétique biocompatible et présente un fond (8) dirigé vers la paroi interne (7) de la sphère d'articulation (5),
**caractérisée en ce que**
le manchon (6) présente la forme d'une coupe et **en ce que**, dans le fond (8), est prévu un évidement (9).

2. Endoprothèse de l'articulation de la hanche selon la revendication 1,
**caractérisée en ce que**
la matière synthétique biocompatible est un polymère.

3. Endoprothèse de l'articulation de la hanche selon la revendication 2,
**caractérisée en ce que**
le polymère est un polyéthylène (PE), polyétheréthercétone (PEEK) ou polyméthylméthacrylate (PMMA).

4. Endoprothèse de l'articulation de la hanche selon l'une des revendications 1 à 3,
**caractérisée en ce que**
le manchon (6) présente la configuration conique d'une coupe.

5. Endoprothèse de l'articulation de la hanche selon l'une des revendications 1 à 4,
**caractérisée en ce que**
le manchon (6) présente un bourrelet circulaire (10).

6. Endoprothèse de l'articulation de la hanche selon la revendication 5,
**caractérisée en ce que**
le bourrelet circulaire (10) est en prise avec une gorge (11) qui est formée sur la paroi interne (7) de la sphère d'articulation (5).

7. Endoprothèse de l'articulation de la hanche selon l'une des revendications 1 à 6,
**caractérisée en ce que**
le manchon (6) est appliqué sur toute sa surface sur la paroi interne (7) de la sphère d'articulation (5) et sur une surface extérieure (12) du cône de la hampe de hanche (4).
